(19) **Europäisches Patentamt**
**European Patent Office**
**Office européen des brevets**

(11) **EP 2 944 376 B1**

(12) **EUROPEAN PATENT SPECIFICATION**

(45) Date of publication and mention
of the grant of the patent:
**13.11.2019 Bulletin 2019/46**

(51) Int Cl.:
**B01J 20/28** *(2006.01)* **B01J 20/26** *(2006.01)*
**C08J 3/16** *(2006.01)*

(21) Application number: **14168064.5**

(22) Date of filing: **13.05.2014**

(54) **Agglomerated superabsorbent polymer particles**

Agglomerierte superabsorbierende Polymerpartikel

Particules de polymère superabsorbant agglomérées

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**18.11.2015 Bulletin 2015/47**

(73) Proprietor: **The Procter & Gamble Company
Cincinnati, OH 45202 (US)**

(72) Inventors:
• **Kamphus, Juliane
65824 Schwalbach (DE)**

• **Lutsche, Marion
65824 Schwalbach (DE)**

(74) Representative: **P&G Patent Germany
Procter & Gamble Service GmbH
Sulzbacher Straße 40
65824 Schwalbach am Taunus (DE)**

(56) References cited:
**WO-A1-2005/012406     WO-A2-2009/095810
US-B2- 7 776 984**

**Description**

FIELD OF THE INVENTION

The present invention is directed to agglomerated superabsorbent polymer particles which have been formed by use of a multivalent salt.

BACKGROUND OF THE INVENTION

The use of superabsorbent polymer particles, especially in absorbent articles, is well known in the art. The superabsorbent polymer particles are typically made by grinding or otherwise shredding relatively large blocks of superabsorbent polymer. However, the size of the particles obtained by such grinding or shredding cannot be fully controlled. The obtained superabsorbent polymer particles hence typically have a certain particle size distribution and hence, there are particles of relatively large size (e.g. 1000 μm or even larger) while others are considerably smaller, such as less than 100 μm or even significantly smaller, with the majority of the particle sizes ranging in between. Small particles are often referred to as "fines".

It is known that in order to have absorbent articles comprising superabsorbent polymer particles which exhibit good absorbing and containing functions, specific technical requirements need to be fulfilled by the superabsorbent polymer particles.

The superabsorbent polymer particles need first to be able to absorb the liquid exudates fast. The absorption speed of superabsorbent polymer particles has generally been characterized in the prior art by measuring the Free Swell Rate (FSR) of the particles.

In addition to having a high absorption speed, the superabsorbent polymer particles present in the absorbent core comprised by absorbent articles need to be also highly permeable to liquid. A poor permeability of the superabsorbent polymer particles may induce leakage of the absorbent article due to gel blocking. Gel blocking can occur in the absorbent core when swelling superabsorbent polymer particles block the void spaces between the particles. In such a case, the liquid exudates cannot or only slowly reach underneath layers of superabsorbent polymer particles disposed in the absorbent core. The liquid exudates remain on the surface of the absorbent core and may therefore leak from the diaper.

The permeability of the superabsorbent polymer particles has typically been characterized in the prior art by measuring the SFC (Saline Flow Conductivity) of the particles. This parameter is measured at equilibrium, i.e. the measure is performed on a fully preswollen gel bed of superabsorbent polymer particles.

To address the problem of gel blocking, superabsorbent polymer particles are typically subjected to surface cross-linking, thus increasing the particle's stiffness and resistance to deformation upon swelling.

While surface cross-linking helps to increase the permeability of the superabsorbent polymer particles, the increase in permeability typically comes at the price of reduced capacity: Given the particles have a stiffer surface, they cannot swell as unhindered as non surface-cross-linked particles. This trade off is more pronounced for smaller superabsorbent polymer particles.

One way of reducing the number of fine particles without having to discard them is to form agglomerates of superabsorbent polymer particles. Though several approaches to form agglomerated superabsorbent polymer particles have been described, there is still a need for an improved method of making agglomerated superabsorbent polymer particles as well as a need for improved agglomerated superabsorbent polymer particles.

US7776984 relates to a process for producing an agglomerated superabsorbent polymer particle and an agglomerated superabsorbent polymer particle. The process comprises the steps of: (A) bringing superabsorbent polymer fine particles having at least about 40 wt. %, a particle size of less than about 150 μm into contact with a fluid comprising to more than about 10 wt.% a cross-linkable, uncrosslinked polymer based on polymerized, ethylenically unsaturated, acid group-bearing monomers or salts thereof; and (B) cross-linking the uncrosslinked polymer by heating the superabsorbent polymer fine particles and the fluid to a temperature from about 20 to about 300°C, so that the cross-linkable, uncrosslinked polymer at least partially crosslinks.

WO2009/095810 relates to absorbent articles that include absorbent compositions which exhibit swelling, deswelling, and reswelling behavior. More specifically, absorbent compositions of this invention swell and absorb fluids after exposure to aqueous fluids, deswell and release fluids from the swollen absorbent compositions, and can also reswell and absorb fluids.

WO2005/012406 relates to a method for producing an agglomerated superabsorber particle, comprising the following steps: (A) fine superabsorber particles, at least 40 percent by weight of which have a particle size of less than 150 μm, are brought into contact with a fluid containing more than 10 percent by weight relative to the total weight of the fluid of a crosslinkable, non-crosslinked polymer, at least 20 percent by weight relative to the total weight of said crosslinkable, non-crosslinked polymer of which are based on polymerized, ethylenically unsaturated, acid group-carrying monomers or the salts thereof; (B) the non-crosslinked polymer is crosslinked by heating the fine superabsorber particles that have been brought into contact with the fluid to a temperature ranging between 20 and 300 °C such that the crosslinkable, non-crosslinked polymer is at least partly crosslinked.

BRIEF DESCRIPTION OF THE DRAWINGS

Fig. 1 is a top view of an exemplary absorbent article in the form of a diaper, which may comprise the agglomerated superabsorbent polymer particles of the present invention, with some layers partially removed.

Fig. 2 is a transversal cross-section of the diaper of Fig. 1.

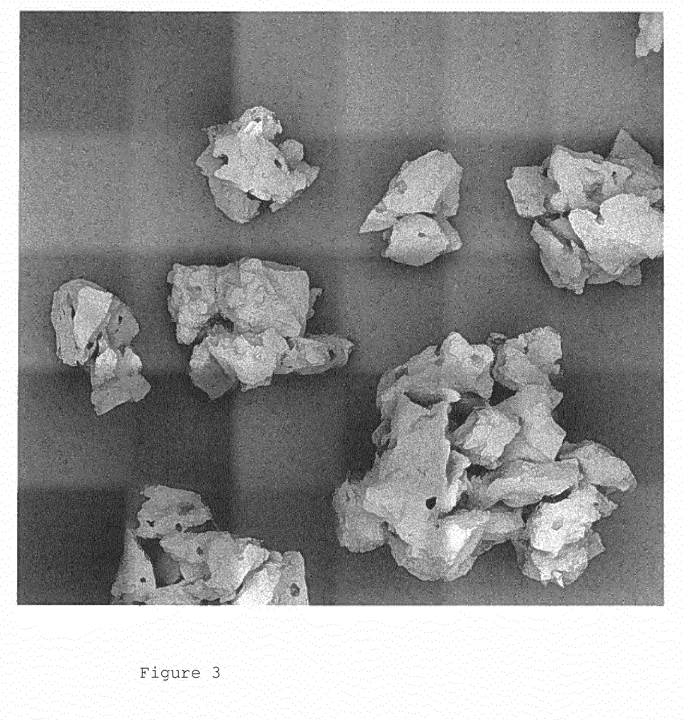

Fig. 3 is a scanning electron micrograph (SEM) of the Example SAP5, which is described below in more detail

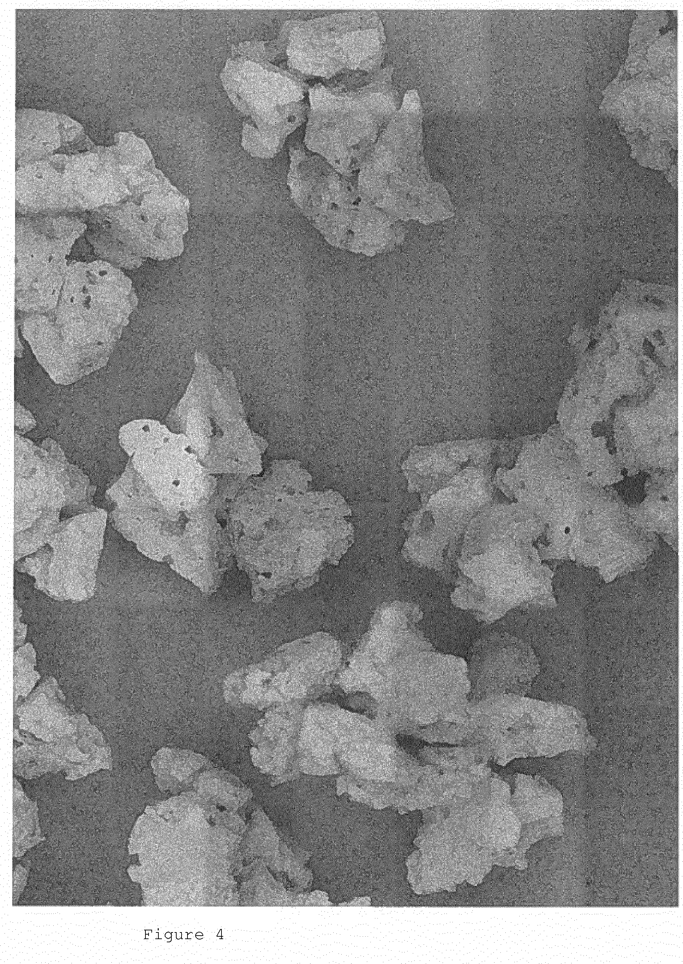

Fig. 4 is a SEM of the Example SAP6, which is described below in more detail.

SUMMARY OF THE INVENTION

Agglomerated superabsorbent polymer particles are disclosed, which are obtained by a method comprising the steps of

a) Providing superabsorbent polymer particles having a first mass average particle size, and

b) mixing the superabsorbent polymer particles with a solution comprising water and a multi-valent salt having a valence of three or higher

The agglomerated superabsorbent polymer particles have a second average particle size which is at least 25% greater than the first mass average particle size.

DETAILED DESCRIPTION OF THE INVENTION

Definitions

"Absorbent article" refers to devices that absorb and contain body exudates, particularly urine and other water-containing liquids, and, more specifically, refers to devices that are placed against or in proximity to the body of the wearer to absorb and contain the various exudates discharged from the body. Absorbent articles may include diapers (baby diapers and diapers for adult incontinence), pants, feminine care absorbent articles such as sanitary napkins or pantiliners, breast pads, care mats, bibs, wipes, and the like. As used herein, the term "exudates" includes, but is not limited to, urine, blood, vaginal discharges, breast milk, sweat and fecal matter. Preferred absorbent articles of the present invention are disposable absorbent articles, more preferably disposable diapers and disposable pants.

"Absorbent core" is used herein to refer to a structure disposed between a topsheet and backsheet of an absorbent article for absorbing and containing liquid received by the absorbent article.

If the absorbent article in addition to the absorbent core comprises a topsheet and/or a backsheet, and/or an acquisition system, the absorbent core does not include the topsheet, the backsheet and/or the acquisition system.

"Airfelt" is used herein to refer to comminuted wood pulp, which is a form of cellulosic fiber.

A "base polymer", as used herein, refers to superabsorbent polymer particles, which have not undergone any surface cross-linking after having been polymerized and grinded into superabsorbent polymer particles.

"Disposable" is used in its ordinary sense to mean an article that is disposed or discarded after a limited number of usage events over varying lengths of time, for example, less than 20 events, less than 10 events, less than 5 events, or less than 2 events. If the disposable absorbent article is a diaper, a pant, sanitary napkin, sanitary pad or wet wipe for personal hygiene use, the disposable absorbent article is most often intended to be disposed after single use.

"Diaper" and "pant" refers to an absorbent article generally worn by babies, infants and incontinent persons about the lower torso so as to encircle the waist and legs of the wearer and that is specifically adapted to receive and contain urinary and fecal waste. In a pant, as used herein, the longitudinal edges of the first and second waist region are attached to each other to a pre-form waist opening and leg openings. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant may be preformed anywhere along the circumference of the article (e.g., side fastened, front waist fastened). In a diaper, the waist opening and leg openings are only formed when the diaper is applied onto a wearer by (releasable) attaching the longitudinal edges of the first and second waist region to each other on both sides by a suitable fastening system.

"Superabsorbent polymer particle" is used herein to refer to cross linked polymeric materials that can absorb at least 10 times their weight of an aqueous 0.9% saline solution as measured using the Centrifuge Retention Capacity test (EDANA WSP 241.2-05). The superabsorbent polymer particles are in particulate form so as to be flowable in the dry state. Preferred superabsorbent polymer particles of the present invention are made of polyacrylic acid polymers. However, e.g. starch-based superabsorbent polymer particles are also comprised within the scope of the present invention

"Thermoplastic adhesive material" is used herein to refer to a polymer composition from which fibers may be formed and applied to the superabsorbent polymer particles with the intent to immobilize the superabsorbent polymer particles in both the dry and wet state. The thermoplastic adhesive material of the present invention preferably forms a fibrous network over the superabsorbent polymer particles.

Superabsorbent polymer particles

The superabsorbent polymer particles useful for forming the agglomerated superabsorbent polymer particles of the present invention may be of numerous shapes. The term "particles" refers to granules, fibers, flakes, spheres, powders, platelets and other shapes and forms known to persons skilled in the art of superabsorbent polymer particles. In some embodiments, the superabsorbent polymer particles can be in the shape of fibers, i.e. elongated, acicular superabsorbent polymer particles. In those embodiments, the superabsorbent polymer particles fibers have a minor dimension (i.e. diameter of the fiber) of less than about 1 mm, usually less than about 500 $\mu$m, and preferably less than 250 $\mu$m down to 50 $\mu$m. The length of the fibers is preferably about 3 mm to about 100 mm.

Alternatively, in some preferred embodiments, superabsorbent polymer particles of the present invention and used for the formation of agglomerated superabsorbent polymer particles are spherical-like particles. According to the present invention and in contrast to fibers, "spherical-like particles" have a longest and a smallest dimension with a particulate ratio of longest to smallest particle dimension in the range of 1-5, where a value of 1 would equate a perfectly spherical particle and 5 would allow for some deviation from such a spherical particle. In such embodiments, the superabsorbent polymer particles may have a particle size of less than 1500 $\mu$m, or less than 1000 $\mu$m, or from 50 to 850 $\mu$m, preferably from 100 to 500 $\mu$m, more preferably from 150 to 300 $\mu$m, as measured according to EDANA method WSP 220.2-05.

The superabsorbent polymer particles useful in the present invention include a variety of water-insoluble, but water-swellable polymers capable of absorbing large quantities of fluids. Such polymers materials are generally known in the art.

Suitable superabsorbent polymer particles may for example be obtained from inverse phase suspension polymerizations as described in U.S. Pat. No. 4,340,706 and U.S. Pat. No. 5,849,816 or from spray- or other gas-phase dispersion polymerizations as described in U.S. Patent Applications No. 2009/0192035, 2009/0258994 and 2010/0068520. In some embodiments, suitable superabsorbent polymer particles may be obtained by current state of the art production processes as is more particularly described from page 12, line 23 to page 20, line 27 of WO 2006/083584.

The surface of the superabsorbent polymer particles may be coated.

Agglomerated superabsorbent polymer particles

The present invention relates to agglomerated superabsorbent polymer particles. It has been surprisingly found that the use of one or more multivalent salts, having a valence of at least three, for making agglomerated superabsorbent polymer particles provides in relatively high yields agglomerated superabsorbent polymer particles with fast initial absorption speed and good permeability while also having good capacity.

Suitable multivalent salts having a valence of at least three are multivalent metal salts. Examples of suitable multivalent metal salts having a valence of three or higher are aluminum sulfate, polyamines, and water-insoluble phosphates of polyvalent metal cations such as zirconium, iron and aluminum. A trivalent cation which can be used in a multivalent salt of the present invention may be a metal cation of the third main group, of the third transition group or of the lanthanide group of the periodic table of the elements, more preferably aluminum, scandium, yttrium, lanthanum or cerium, most preferably aluminum.

Suitable trivalent cations are the cations of aluminum, iron, chromium, rare earths and manganese, suitable tetravalent cations are cations of titanium and zirconium. Possible counterions are chloride, bromide, sulfate, hydrogensulfate, carbonate, hydrogencarbonate, nitrate, phosphate, hydrogenphosphate, dihydrogenphosphate and carboxylate, such as acetate and lactate. Among those, aluminum sulfate and aluminum lactate are preferred. It is also possible to use polyamines as further polyvalent cations.

Generally, for the present invention, preferred multivalent salts having a valence of three or higher may comprise aluminum and may further comprise lactate. These include aluminum lactate but also mixed salts with aluminum salts and other lactates.

The use of multivalent salts having a valence of at least three improves the formation of agglomerated superabsorbent polymer particles compared to agglomerate formation in the presence of water. Without wishing to be bound by theory, it is believed that the cations of the multivalent salt can to a certain degree substitute the sodium cations, which are typically present inter alia at the surface of superabsorbent polymer particles. Due to their higher valence compared to the monovalent sodium cation, the multivalent cation can establish ionic interactions with more than one superabsorbent polymer particle, thus "bridging" neighboring superabsorbent polymer particles. Thus, relatively strong ionic interactions ("ionic bonds") are formed between superabsorbent polymer particles within the agglomerated superabsorbent polymer particles compared to the relatively weak hydrogen bonds formed if agglomeration is done only in the presence of water. Agglomerates are formed more efficiently when using multivalent salts having a valence of at least three, because, during formation of agglomerates, the agglomerates tend to remain in their agglomerated state more readily compared to agglomeration in the presence of only water, where agglomerates already formed may separate and "fall apart" again, hence not forming stable agglomerates.

The resulting agglomerated superabsorbent polymer particles are believed to be more stable in the presence of water as well as in the presence of bodily liquids such as urine due to the ionic bonds between the superabsorbent polymer particles (versus weaker hydrogen bonds formed in agglomerated superabsorbent polymer particles formed in the presence of water only).

Also, agglomerated superabsorbent polymer particles of the present invention allow for less compacted agglomerates versus those formed by hydrogen bonds due to higher bond strength between the particles: Higher bond strength enables less contact area between the agglomerated superabsorbent polymer particles while still providing stable agglomerates. By way of comparison, the reduced contact area sufficient to form stable agglomerates is similar to the use of relatively strong glue to bond different surfaces to each other. Stronger glues can be provided on a smaller surface area versus weaker glue where higher surface area coverage is required to obtained comparable bond strength.

The reduced contact area and reduced compaction results in more surface area of the agglomerated superabsorbent polymer particles to absorb liquid in use, e.g. when the agglomerated superabsorbent polymer particles are used in absorbent articles. This, in turn, leads to agglomerated superabsorbent polymer particles with relatively high speed of absorption, which may be especially desirable in initial fluid uptake of agglomerated superabsorbent polymer particles, as is reflected by low uptake times given as T20 values as is explained in more detail below.

For completeness, agglomerated superabsorbent polymer particles wherein the superabsorbent polymer particles are bound to each other by covalent bonds may suffer from a reduced capacity: Upon swelling of agglomerated superabsorbent polymer particles, the polymer chains within a particle tend to "move" and slightly rearrange their position. As covalent bonds cannot be easily opened and re-formed, such rearrangement of polymer chains may create tension which may either lead to reduced swelling of the particles (hence, leading to reduced capacity), or the covalent bonds may break. Once broken, since reformation is not readily possible, the stability of the agglomerated superabsorbent polymer particles may be adversely affected. Compared thereto, ionic interactions can be more readily reversed and re-formed. Upon swelling of the agglomerated superabsorbent polymer particles, the ionic bond may hence move slightly within a given polymer chain (i.e. on a molecular level) such that the ionic bond forms again at a position adjacent to the initial ionic bond. Thereby neither the capacity nor the stability of the agglomerated superabsorbent polymer particles are impaired.

The agglomerated superabsorbent polymer particles of the present invention may have a time to reach an uptake of 20 g/g (T20) of less than 240s, or less than 200 s, or less than 180s. The time to reach an uptake of 20 g/g (T20) may be at least 40s, or at least 60 s, or at least 80 s, or at least 100 s. T20 is measured according to the K(t) test method described in EP2535027 A1.

Absorbent articles comprising agglomerated superabsorbent polymer particles with low uptake times, have improved absorption properties and therefore exhibit reduced leakage in comparison with absorbent articles of the prior art, especially at the first gush (i.e. first liquid insult). Hence, such superabsorbent polymer particles are particularly suitable for use in absorbent articles.

The agglomerated superabsorbent polymer particles of the present invention may have an UPM value of from $40.10^{-7}$ cm$^3$.s/g to $500.10^{-7}$ cm$^3$.s/g, or from $50.10^{-7}$ cm$^3$.s/g to $400.10^{-7}$ cm$^3$.s/g, or from $80.10^{-7}$ cm$^3$.s/g to $400.10^{-7}$ cm$^3$.s/g

The UPM value is measured according to the UPM Test method set out in EP2535027 A1. This method is closely related to the SFC test method of the prior art. The UPM Test method measures the flow resistance of a preswollen layer of superabsorbent polymer particles, i.e. the flow resistance is measured at or close to equilibrium. Therefore, agglomerated superabsorbent polymer particles having a high UPM value exhibit a high permeability- High permeability is especially desirable in absorbent articles, when a significant volume of the absorbent article is already wetted by the liquid exudates. These absorbent articles exhibit good absorption properties not only at the first gush but also at the subsequent gushes.

Moreover, the agglomerated superabsorbent polymer particles may have a capacity, determined as Centrifuge Retention Capacity (CRC) of from 18 g/g to 40 g/g, or from 22 g/g to 40 g/g.

The CRC measures the liquid absorbed by the superabsorbent polymer particles for free swelling in excess liquid. Superabsorbent polymer particles having relatively high CRC value are preferred since less superabsorbent polymer particles are needed to facilitate a required overall capacity for liquid absorption.

The agglomerated superabsorbent polymer particles of the present invention can be made as follows:

a) *Superabsorbent polymer particles are provided having a first mass average particle size:*

Further, for purposes of this invention, the mass average particle size of the superabsorbent polymer particles (for the first mass average particle size) or the agglomerated superabsorbent polymer particles (for the second mass average particle size) is defined as the particle size which is the average particle size of the given superabsorbent polymer particles (or agglomerated superabsorbent polymer particles) on a mass basis. A method for determining the mass average particle size is described hereinafter in the Test Methods section. The mass average particle size of the superabsorbent polymer particles (or agglomerated superabsorbent polymer particles) may be from 50 $\mu$m to 850 $\mu$m, or from 100 $\mu$m to 650 $\mu$m, or from 200 $\mu$m to 600 $\mu$m as long as the second mass average particle size is at least 25% higher than the first mass average particle size.

The superabsorbent polymer particles having the first mass average particle size may be any superabsorbent polymer particles. However, it may be desirable to use superabsorbent polymer particles which have been classified. For example, the superabsorbent polymer particles may have been classified such that they have a defined an upper particle size limit. The upper particle size limit may be 500 $\mu$m, or 400 $\mu$m, or 300 $\mu$m, or 150 $\mu$m.

Classification is well known in the art and is typically done by sieving the particles using a defined mesh size. The particles passing the sieve will have a particle size lower than the mesh size used while the particles not passing through the sieve will (mostly) have a particle size equal to or larger than the mesh size. As will be understood, if too many superabsorbent polymer particles are sieved at the simultaneously, some of the superabsorbent polymer particles not passing through the sieve may nevertheless have a particle size smaller than the mesh size. Hence, as is understood by the skilled person, it is important to carefully and diligently carry out the sieving, not using too many superabsorbent polymer particles at a time.

Moreover, the shape of the superabsorbent polymer particles or agglomerated superabsorbent polymer particles to be sieved also impacts the accurateness of sieving. For particles or agglomerates having very irregular shape, their current orientation may determine whether or not they pass through a given mesh size. Hence, sieving superabsorbent polymer particles or agglomerated superabsorbent polymer particles with very irregular shape may lead to a relatively high standard deviation. As will be understood by the skilled person, in such circumstances it may be desirable to increase the repetitions of sieving or increase the number of samples, accordingly.

Hence, the upper and lower particle size limits as used herein, given they are obtained by classification using sieving, are to be understood as follows: For upper particle size limits, the respective superabsorbent polymer particles or the agglomerated superabsorbent polymer particles may comprise up to 10%, or up to 5% by weight of superabsorbent polymer particles or the agglomerated superabsorbent polymer particles having a higher particle size than the upper particle size limit. For lower particle size limits, the respective superabsorbent polymer particles or the agglomerated superabsorbent polymer particles may comprise up to 10%, or up to 5% by weight of superabsorbent polymer particles or the agglomerated superabsorbent polymer particles having a lower particle size than the lower particle size limit.

Additionally to classifying the superabsorbent polymer particles to have an upper particle size limit, it may also be desirable to further classify the superabsorbent polymer particles such that they also have a lower particle limit. The lower particle size limit may be 20 $\mu$m, or 30 $\mu$m, or 50 $\mu$m, or 80 $\mu$m.

The difference between the upper particle size limit and the lower particle size limit of the superabsorbent polymer particles provided in step a) may be less than 600 $\mu$m, or less than 500 $\mu$m, or less than 300 $\mu$m, or less than 150 $\mu$m. The difference between the upper particle size limit and the lower particle size limit may also not be less than 20 $\mu$m.

Classification can be used to obtain superabsorbent polymer particles having the desired first mass average particle size.

The superabsorbent polymer particles provided may be base polymer, i.e. superabsorbent polymer particles not having undergone any surface cross-linking. However, although less desirable, the superabsorbent polymer particles may also be surface cross-linked and/or may have undergone other treatments, such as coating.

Generally, the superabsorbent polymer particles provided under step a) are desirable dry superabsorbent polymer particles having a water content of less than 10% by weight, or less than 5% by weight, or less than 3% by weight of the superabsorbent polymer particles. However, the superabsorbent polymer particles provided under a) may also have a higher water content, such as up to 15% by weight, up to 25% by weight, up to 50% by weight, or even higher.

*b) Mixing the superabsorbent polymer particles with a solution comprising water and a multi-valent salt having a valence of three or above.*

The superabsorbent polymer particles having a first mass average particle size are mixed with a solution comprising water and a multivalent salt having a valence of three or higher. The solution may comprise further solvents in addition to water, such as organic solvents, however, it is desirable not to use any solvents in addition to water.

Mixing of the superabsorbent polymer particles with the solution comprising water and the multivalent salt having a valence of three or higher may be done at a rate of at least 1 g, or at least 2 g, or at least 4 g, or at least 6 g, or at least 8 g of solution per kg of provided superabsorbent polymer particles per minute. The rate may be less than 200 g/kg/min, or less than 100 g/kg/min.

The solution comprises from 3% to 80% by weight of the multivalent salt having a valence of three or higher, such as from 5% to 50%, or from 10% to 30%, or from 12% to 25%.

Mixing can be done by spraying the solution comprising water and the multivalent salt having a valence of three or higher onto the superabsorbent polymer particles. It may be desirable to agitate the superabsorbent polymer particles during and/or after the solution is applied. Generally, the multivalent salt having a valence of three or higher should be applied onto the superabsorbent polymer particles relatively homogeneously.

Mixing can be done in equipment well known in the art, such as coaters, paddle mixers, ploughshare mixers, kneaders, fluidized bed coaters, Wurster coaters, spinning disk reactors, etc.

Mixing can be done at room temperature or at elevated temperatures (e.g. around 70°C to 100 °C). If mixing is done at elevated temperatures, the resulting agglomerated superabsorbent polymer particles may already be sufficiently dry such that no subsequent drying step is needed. However, the temperatures may not be as high as to evaporate an excessive amount of the applied solution too fast.

If desired, mixing can be followed by a dying step, e.g. at temperatures of e.g. 70°C to 100 °C.

The agglomerated superabsorbent polymer particles obtained by step b) and the optional drying step are desirably dry agglomerated superabsorbent polymer particles. The dry agglomerated superabsorbent polymer particles may have a water content of less than 10% by weight, or less than 5% by weight, or less than 3% by weight.

According to the present invention, the mass average particle size of the obtained agglomerated superabsorbent polymer particles (herein referred to as the second mass average particle size) is at least 25% higher than the first mass average particle size, which is the mass average particle size of the superabsorbent polymer particles before having been subjected to the solution comprising water and the multivalent salt having a valence of three or higher. The second mass average particle size may be at least 30%, or at least 40% or at least 50% higher than the first mass average particle size.

If the second mass average particle size, after step b) has been carried out, is less than 25% higher than the first mass average particle size, a classification step can be added after step b).

By this classification step, the amount of non-agglomerated superabsorbent polymer particles can be significantly reduced such that the second mass average particle size increases.

The optional classification step carried out after step b), and after the optional drying step eliminates superabsorbent polymer particles with a particle size below a lower particle size limit. The lower particle size limit of the optional classification step and hence, of the agglomerated superabsorbent polymer particles may be 150 $\mu$m, or may be 300 $\mu$m, or may be 500 $\mu$m.

If the superabsorbent polymer particles having the first mass average particle size have been provided by a classification step and have an upper particle size limit, the lower particle size limit of the optional classification step carried out after step b) (and after the optional drying step), the lower particle size limit of the classification step on the agglomerated superabsorbent polymer particles may be from 80% to 100%, or from 90% to 100% of the upper particle size limit of the superabsorbent polymer particles having a first mass average particle size. Hence, in this latter classification step, the majority (or substantially all, if the lower particle size limit of the agglomerates is 100% of -i.e. equal to - the upper particle size of the particles with the first average particle size) of non-agglomerated superabsorbent polymer particles will be sieved out and the agglomerated superabsorbent polymer particles are retained.

If the agglomerated superabsorbent polymer particles of the present invention are formed with base polymer particles, i.e. with superabsorbent polymer particles not having undergone any surface cross-linking prior to agglomerate formation, the base polymer made be obtained as follows:

- Providing a polyacrylic acid polymer gel. The polyacrylic acid polymer gel may be made by well known methods, and may e.g. be made by polymerizing acrylic acid monomers, e.g. by polymerizing the acrylic acid monomers at 50% to 95% neutralization. Neutralization is typically done with sodium hydroxide.

- The gel is submitted to a first grinding step followed by a first drying step to obtain base polymer particles.

- If, after the first drying step, the base polymer particles are too dry for the second grinding step, the base polymer particles can optionally be rewetted.

- Subsequently, the base polymer particles may be subjected to a second grinding step followed by a second drying step.

Before being submitted to the second grinding step, the water content of the base polymer particles may be at least 0.4 g water per g of dry base polymer particles, but may be not more than 15 g/g, desirably from 0.4 g/g to 5 g/g, or from 0.8 g/g to 3 g/g, or from 1 g/g to 2 g/g. The respective water content can be obtained by either drying the base polymer particles in the first drying step to the desired water content level (i.e. not "completely" drying the base polymer particles). Alternatively, if the base polymer particles derived after the first drying step are too dry, they can be rewetted to obtain the desired water content prior to the second grinding step.

By providing a first and a second grinding step, it is possible to obtain base polymer particles having a relatively high surface area as the polymer has been exposed to the shear forces in the grinder two times.

A detailed example of a base polymer is provided below.

Surface cross-linking

Surface cross-linking of superabsorbent polymer particles is well known in the art. For the present invention, surface cross-linking of the agglomerated superabsorbent polymer particles or, though less desirable, surface cross-linking of the superabsorbent polymer particles prior to or simultaneously with formation of the agglomerates can be done by any of the known surface cross-linking methods.

Commonly applied surface cross-linkers are thermally activated surface cross-linkers. The term "thermally activated surface cross-linkers" refers to surface cross-linkers, which only react upon exposure to increased temperatures, typically around 150 °C. Thermally activated surface cross-linkers known in the prior art are e.g. di- or polyfunctional agents that are capable of building additional cross-links between the polymer chains of the superabsorbent polymer particles. Other thermally activated surface cross-linkers include, e.g., di- or polyhydric alcohols, or derivatives thereof, capable of forming di- or polyhydric alcohols. Representatives of such agents are alkylene carbonates, ketales, and di- or polyglycidlyethers. Moreover, (poly)glycidyl ethers, haloepoxy compounds, polyaldehydes, polyoles and polyamines are also well known thermally activated surface cross-linkers. The cross-linking is based on a reaction between the functional groups comprised by the superabsorbent polymer particle, for example, an esterification reaction between a carboxyl group (comprised by the polymer) and a hydroxyl group (comprised by the surface cross-linker).

In general, the surface cross-linking agent is applied on the surface of the superabsorbent polymer particles prior to, during, or, more desirable, after the agglomerated superabsorbent polymer particles are formed due to mixing the superabsorbent polymer particles with the solution comprising water and a multivalent salt having a valence of three or higher. Therefore, the reaction preferably takes place on the surface of the superabsorbent polymer particles or of the agglomerated superabsorbent polymer particles, which results in improved cross-linking on the surface of the particles while not substantially affecting the core of the particles. Thereby, the surface of the (agglomerated) superabsorbent polymer particles becomes stiffer.

Surface cross-linking agents are often applied in a solution which consists of, or comprises an organic solvent. Such organic solvent generally renders the surface of the superabsorbent polymer particles less sticky compared to the use of water as solvent. However, for forming agglomerated superabsorbent polymer particles, a sticky surface is desirable as it fosters agglomeration. Hence, especially if the surface cross-linkers are applied in a solution consisting of or comprising an organic solvent, it may be desirable to surface cross-link the superabsorbent polymer particles after the agglomerated superabsorbent polymer particles are formed due to mixing the superabsorbent polymer particles with the solution comprising water and a multivalent salt having a valence of three or higher.

However, the surface cross-linker can also be added in an aqueous solution, via gas phase (i.e. vaporizing a liquid surface cross-linker) or by adding a pure surface cross-linker in liquid form.

The surface cross-linker may also be added together with other substances, such as surfactants.

Typically, surface cross-linking will be done at temperatures of at least 100°C, or at least 120°C, or at least 150°C. It may be desirable to not increase the temperature above 200°c, or not above 180°C to avoid e.g. yellowing of the (agglomerated) superabsorbent polymer particles.

Absorbent articles

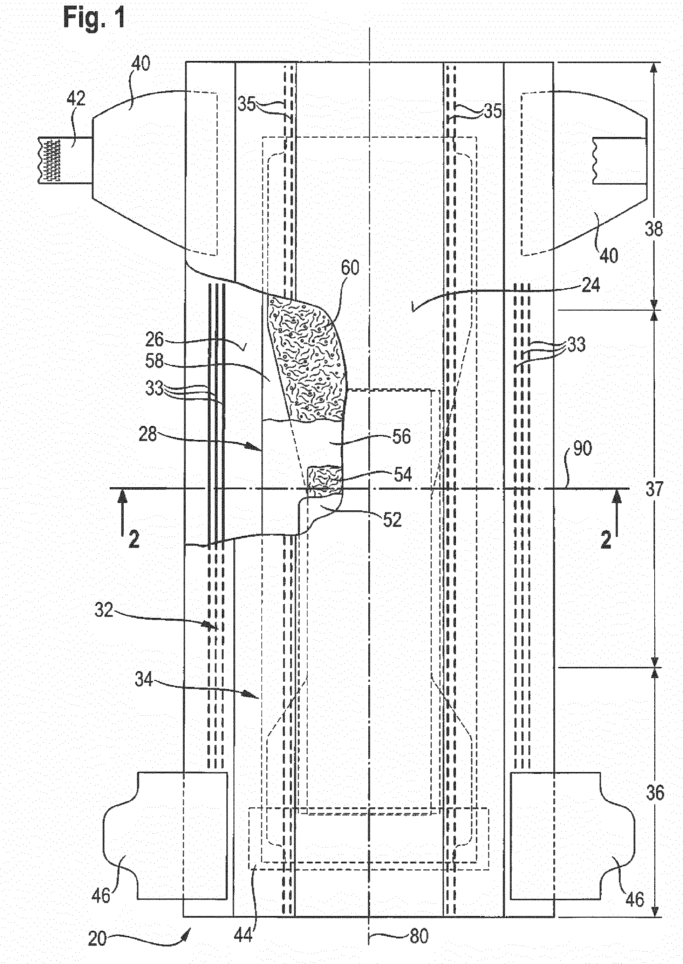

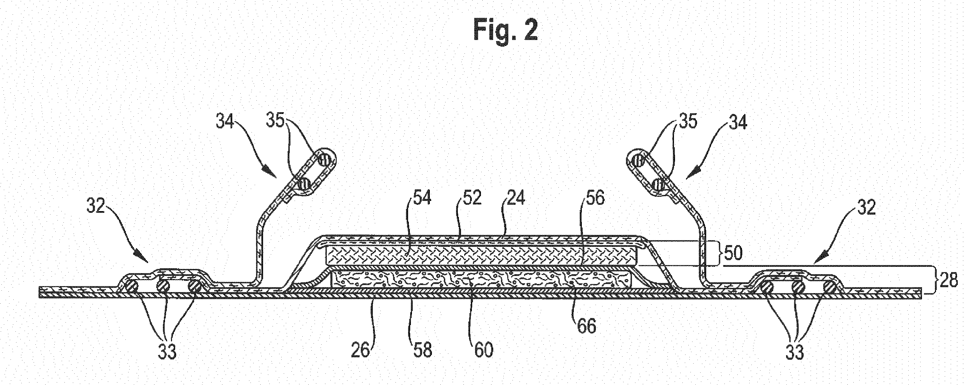

A typical disposable absorbent article, in which the agglomerated superabsorbent polymer particles of the present invention can be used, is placed against or in proximity to the body of the wearer to absorb and contain the various exudates discharged from the body and is represented in Fig. 1 and 2 in the form of a diaper 20.

In more details, Fig. 1 is a plan view of an exemplary diaper 20, in a flat-out state, with portions of the diaper being cut-away to more clearly show the construction of the diaper 20. This diaper 20 is shown for illustration purpose only as the structure of the present invention may be comprised in a wide variety of diapers or other absorbent articles.

As shown in Fig. 1 and 2, the absorbent article, here a diaper, can comprise a liquid pervious topsheet 24, a liquid impervious backsheet 26, an absorbent core 28 which is positioned between f the topsheet 24 and the backsheet 26. The absorbent core 28 can absorb and contain liquid received by the absorbent article and may comprise absorbent materials 60, such as the agglomerated superabsorbent polymer particles of the present invention 66 and/or cellulose fibers, as well as other absorbent and non-absorbent materials commonly used in absorbent articles (e.g. thermoplastic adhesives immobilizing the superabsorbent polymer particles). The absorbent material and non-absorbent material may be wrapped within a substrate (e.g. one or more nonwovens, tissues etc.) such as by an upper core cover layer 56 facing towards the topsheet and a lower cover layer 58 facing towards the backsheet. Such upper and lower core cover layers may be made of nonwovens, tissues or the like and may be attached to each other continuously or discontinuously, e.g. along their perimeter

The absorbent core may comprise one or more substrate layer(s) (such as nonwoven webs or paper tissue), superabsorbent polymer particles disposed on the one or more substrate layers, and a thermoplastic composition typically disposed on the superabsorbent polymer particles. Typically the thermoplastic composition is a thermoplastic adhesive material. In one embodiment, the thermoplastic adhesive material forms a fibrous layer which is at least partially in contact with the superabsorbent polymer particles on the one or more substrate layers and partially in contact with the one or more substrate layers. Auxiliary adhesive might be deposited on the one or more substrate layers before application of the superabsorbent polymer particles for enhancing adhesion of the superabsorbent polymer particles and/or of the thermoplastic adhesive material to the respective substrate layer(s). The absorbent core may also include one or more cover layer(s) such that the superabsorbent polymer particles are comprised between the one or more substrate layer(s) and the one or more cover layer(s). The one or more substrate layer(s) and the cover layer(s) may comprise or consist of a nonwoven web. The absorbent core may further comprise odor control compounds.

The absorbent core may consist essentially of the one or more substrate layer(s), the superabsorbent polymer particles, the thermoplastic composition, optionally the auxiliary adhesive, optionally the cover layer(s), and optionally odor control compounds.

The absorbent core may also comprise a mixture of superabsorbent polymer particles and airfelt, which may be enwrapped within one or more substrate layers, such as nonwoven webs or paper tissue. Such absorbent cores may comprise from 30% to 95%, or from 50% to 95% of superabsorbent polymer particles by weight of the absorbent material and may comprise from 5% to 70%, or from 5% to 50% of airfelt by weight of the absorbent material (for these percentages, any enwrapping substrate layers are not considered as absorbent material). The absorbent core may also be free of airfelt and may comprise 100% of superabsorbent polymer particles by weight of the absorbent material.

The absorbent core may comprise mixtures of the agglomerated superabsorbent polymer particles of the present invention and other superabsorbent polymer particles. For example, the absorbent core may comprise at least 70%, or at least 80%, or at least 90% or 100% of superabsorbent polymer particles by weight of the absorbent material, wherein the superabsorbent polymer particles comprise at least 10%, or at least 20% or at least 30% or at least 50% by weight of the agglomerated superabsorbent polymer particles.

The absorbent articles of the invention, especially diapers and pants, may comprise an acquisition layer 52, a distribution layer 54, or combination of both (all herein collectively referred to as acquisition-distribution system "ADS" 50). The function of the ADS 50 is typically to quickly acquire the fluid and distribute it to the absorbent core in an efficient manner. The ADS may comprise one, two or more layers. In the examples below, the ADS 50 comprises two layers: a distribution layer 54 and an acquisition layer 52 disposed between the absorbent core and the topsheet.

The ADS may be free of superabsorbent polymer. The prior art discloses many types of acquisition-distribution systems, see for example WO2000/59430, WO95/10996, US5700254, WO02/067809. However, the agglomerated superabsorbent polymer particles of the present invention may also be comprised by the ADS.

The function of a distribution layer 54 is to spread the insulting fluid liquid over a larger surface within the article so that the absorbent capacity of the absorbent core can be more efficiently used. Distribution layers may be made of a nonwoven material based on synthetic or cellulosic fibers and having a relatively low density. The distribution layer may typically have an average basis weight of from 30 to 400 g/m$^2$, in particular from 80 to 300 g/m$^2$.

The distribution layer may for example comprise at least 50%, or 60%, or 70%, or 80%, or 90% by weight of cross-linked cellulose fibers. The cross-linked cellulosic fibers may be crimped, twisted, or curled, or a combination thereof including crimped, twisted, and curled. The cross-linked cellulosic fibers provide higher resilience and therefore higher resistance to the first absorbent layer against the compression in the product packaging or in use conditions, e.g. under baby weight. This provides the core with a relatively high void volume, permeability and liquid absorption, and hence reduced leakage and improved dryness.

The distribution layer comprising cross-linked cellulose fibers, may comprise other fibers, but this layer may advantageously comprise at least 50%, or 60%, or 70%, or 80%, or 90% or even up to 100%, by weight of the layer, of cross-linked cellulose fibers. Examples of such mixed layer of cross-linked cellulose fibers may comprise 70% by weight of chemically cross-linked cellulose fibers, 10 % by weight polyester (PET) fibers, and 20 % by weight untreated pulp fibers. In another example, the layer of cross-linked cellulose fibers may comprise 70 % by weight chemically cross-linked cellulose fibers, 20 % by weight lyocell fibers, and 10% by weight PET fibers. In another example, the layer may comprise 68 % by weight chemically cross-linked cellulose fibers, 16 % by weight untreated pulp fibers, and 16 % by weight PET fibers.

The absorbent article 20 may further comprise an acquisition layer 52, whose function is to quickly acquire the fluid away from the topsheet so as to provide a good dryness for the wearer. The acquisition layer 52 is typically placed directly under the topsheet and below the distribution layer. The acquisition layer may typically be or comprise a non-woven material, for example a SMS or SMMS material, comprising a spunbonded, a melt-blown and a further spunbonded layer or alternatively a carded chemical-bonded nonwoven. The non-woven material may in particular be latex bonded. Exemplary upper acquisition layers 52 are disclosed in US7786341. Carded, resin-bonded nonwovens may be used, in particular where the fibers used are solid round or round and hollow PET staple fibers (such as a 50/50 or 40/60 mix of 6 denier and 9 denier fibers). An exemplary binder is a butadiene/styrene latex.

The acquisition layer 52 may be stabilized by a latex binder, for example a styrene-butadiene latex binder (SB latex). Processes for obtaining such lattices are known, for example, from EP 149 880 (Kwok) and US 2003/0105190 (Diehl et al.). The binder may be present in the acquisition layer 52 in excess of 12%, 14% or 16% by weight, but may be present by not more than 30%, or not more than 25% by weight of the acquisition layer. SB latex is available under the trade name GENFLO™ 3160 (OMNOVA Solutions Inc.; Akron, Ohio).

A further acquisition layer may be used in addition to a first acquisition layer described above. For example a tissue layer may be placed between the first acquisition layer and the distribution layer. The tissue may have enhanced capillarity distribution properties compared to the acquisition layer described above. The tissue and the first acquisition layer may be of the same size or may be of different size, for example the tissue layer may extend further in the back of the absorbent article than the first acquisition layer. An example of hydrophilic tissue is a 13 - 15 gsm high wet strength made of cellulose fibers from supplier Havix.

The diaper may also comprise elasticized leg cuffs 32 and barrier leg cuffs 34, which provide improved containment of liquids and other body exudates especially in the area of the leg openings. Usually each leg cuffs 32 and barrier cuffs 34 will comprise one or more elastic string 33 and 35, represented in exaggerated form on Figs. 1 and 2. Moreover, the diaper 20 may comprise other features such as back ears 40, front ears 46 and/or barrier cuffs 34 attached to form the composite diaper structure. The diaper may further comprise a fastening system, such as an adhesive fastening system or a mechanical fastening system (e.g. a hook and loop fastening system), which can comprise tape tabs 42, such as adhesive tape tabs or tape tabs comprising hook elements, cooperating with a landing zone 44 (e.g. a nonwoven web providing loops in a hook and loop fastening system). Further, the diaper may comprise other elements, such as a back elastic waist feature and a front elastic waist feature, side panels or a lotion application.

The diaper 20 as shown in Figures 1 and 2 can be notionally divided in a first waist region 36, a second waist region 38 opposed to the first waist region 36 and a crotch region 37 located between the first waist region 36 and the second waist region 38. The longitudinal centerline 80 is the imaginary line separating the diaper along its length in two equal halves. The transversal centerline 90 is the imagery line perpendicular to the longitudinal line 80 in the plane of the flattened out diaper and going through the middle of the length of the diaper. The periphery of the diaper 20 is defined by the outer edges of the diaper 20. The longitudinal edges of the diaper may run generally parallel to the longitudinal centerline 80 of the diaper 20 and the end edges run between the longitudinal edges generally parallel to the transversal centerline 90 of the diaper 20.

TEST METHODS

Mass Average Particle Size via Sieve Test

10 g (weighed to an accuracy of at least 0.01 g) of a representative sample of the respective superabsorbent polymer particles or agglomerated superabsorbent polymer particles are sieved via sieves of about 10 cm in diameter (available e.g. from Retsch GmbH, Haan, Germany; DIN/ISO 3310-1). A stack of sieve with the following mesh sizes (sequence from top to bottom) is used: 850 $\mu$m, 800 $\mu$m, 710 $\mu$m, 600 $\mu$m, 500 $\mu$m, 425 $\mu$m, 300 $\mu$m, 212 $\mu$m, 150 $\mu$m, pan (taken herein as equivalent to 0 $\mu$m). The weight of each empty sieve is noted down, to an accuracy of 0.01 g.

The 10 g sample is loaded to the top sieve (i.e. 850 $\mu$m) and sieved via a sieve machine ("AS 400 control" available from Retsch GmbH, Haan, Germany) for 3 min at 250 rpm. The weight of each sieve after sieving is noted down, to an accuracy of 0.01 g. The difference between the weight of loaded sieve and the empty sieve for each size gives the weight of particles per mesh size.

As size of the sieve $D_i$ the sieve notation is taken, e.g. on sieve 500 $\mu$m is the fraction with D500 to an amount of m500, with D500 = 500 $\mu$m.

The mass average particle size (mAvPS) herein is calculated as

$$mAvPS = \frac{\sum_i m_i \cdot D_i}{m_{total}}$$

with

$$m_{total} = \sum_i m_i$$

The K(t) Test Method (Dynamic Effective Permeability and Uptake Kinetics Measurement Test method) for measuring T(20) and U20, as well as the UPM and FSR (Free Swell Rate) test method are disclosed in detail in EP2535027A1. For the Examples herein, the K(t) and UPM test methods disclosed in EP2535027A1 were followed. The test methods were carried out, using the agglomerated superabsorbent polymer particles of the present invention.

The K(t) Test Method determines the time dependent effective permeability (K(t)) and the uptake kinetics of a gel layer formed from hydrogel-forming superabsorbent polymer particles or of an absorbent structure containing such particles under a confining pressure. For the present invention, the K(t) test method is applied, using the agglomerated superabsorbent polymer particles (not comprised in an absorbent structure). The objective of this method is to assess the ability of the gel layer formed from hydrogel-forming superabsorbent polymer particles to acquire and distribute body fluids when the polymer is present at high concentrations in an absorbent article and exposed to mechanical pressures as they typically occur during use of the absorbent article. Darcy's law and steady-state flow methods are used to calculate effective permeability. (See also for example, "Absorbency," ed. by P.K. Chatterjee, Elsevier, 1982, Pages 42-43 and "Chemical Engineering Vol. II, Third Edition, J.M. Coulson and J.F. Richardson, Pergamon Press, 1978, Pages 122-127.)

In contrast to other known, published methods, the sample in the K(t) test method is not preswollen, therefore the hydrogel is not formed by preswelling hydrogel-forming superabsorbent polymer particles in synthetic urine, but the measurement is started with dry particles.

The UPM value is measured according to the UPM Test method set out in EP2535027A1 And closely related to the SFC (Saline Flow Conductivity) test method of the prior art. The UPM Test method typically measures the flow resistance of a preswollen layer of superabsorbent polymer particles, i.e. the flow resistance is measured at equilibrium. Therefore, such superabsorbent polymer particles having a high UPM value exhibit a high permeability when a significant volume of the absorbent article is already wetted by liquid exudates.

CRC (Centrifuge Retention Capacity) has been measured according to EDANA method WSP 241.2-05.

EXAMPLES

Preparation of Base Polymer A:

A 20 000 ml resin kettle (equipped with a four-necked glass cover closed with septa, suited for the introduction of a thermometer, syringe needles) is charged with about 5089.0 g of ice (representing about 30-40% of the total amount of ice, which is 12128.0 g; the ice being prepared from deionized water). A magnetic stirrer, capable of mixing the whole content (when liquid), is added and stirring is started.

45.7 g of deionized water is taken to dissolve 4.516 g of "V50" (= 2,2'-azobis (N,N'-dimethyleneisobutyramidine) dihydrochloride, available from Wako Chemicals, Neuss, Germany) e.g. in a glass vessel with plastic snap-on cap. The vessel with the "V50" solution is closed and set aside in a fridge at about 4°C.

312.5 g of glacial acrylic acid (herein after referred to as "AA"; e.g. Acrylic Acid for synthesis, available from Merck KGaA, Germany) is taken from the total amount of 4000.1 g AA to dissolve 25.67 g of MethyleneBisAcrylAmid (hereinafter referred to as "MBAA") e.g. in a glass beaker. The beaker with the MBAA solution is covered e.g. with parafilm and set aside.

The remaining AA is added to the ice in the resin kettle while stirring is continued.

A thermometer is introduced and in total 3330.7 g of 50% NaOH solution (for analysis, available from Merck KGaA, Germany) and the remaining amount of ice (prepared from deionized water) are added subsequently in portions such that the temperature remains in the range of about 15-30°C.

The MBAA solution is added to the mixture of AA, NaOH solution and ice at a temperature of about 15-30°C while stirring is continued. The beaker containing the MBAA solution is washed two times with deionized water in an amount of about 10% of the MBAA solution volume per wash. The wash water of both washing steps is added to the stirred mixture.

Deionized water (the remaining amount required to achieve the total amount (ice and water) of 12639.3 g minus the amount to wash the "V50" containing vessel two times with deionized water in an amount of about 10% of the "V50" solution volume per wash) is added to the stirred mixture.

Then, the resin kettle is closed, and a pressure relief is provided e.g. by puncturing two syringe needles through the septa. The solution is then purged vigorously with argon via a 80 cm injection needle at about 0.4 bar while stirring at about 400 - 1200 RPM. The argon stream is placed close to the stirrer for efficient and fast removal of dissolved oxygen.

After a minimum of one hour and a maximum of two hours of argon purging and stirring, the "V50" solution is added to the reaction mixture at a temperature of about 20-25°C via a syringe while stirring and Argon purging is continued. The vessel containing the "V50" solution is washed two times with deionized water in an amount of about 10% of the "V50" solution volume per wash. The wash water of both washing steps is added to the stirred mixture via a syringe through the septa.

After the initiator solution is mixed with the reaction mixture, stirring and argon purging is continued for about 5 min. After that, while the reaction mixture has a temperature of about 20-25°C, two photo lamps ("ProVision 2.55 HF" available from Kaiser Fototechnik GmbH & Co.KG, Buchen, Germany, equipped with 2 lamps Osram Dulux L 55W/830, at max. intensity) are placed on either side of the vessel and switched on. The solution typically starts to become turbid or a sudden increase in viscosity is observed after about 5-20 min, typically at temperatures about room temperature. Then, the argon injection needle is raised above the surface of the gel and purging with argon is continued at a reduced flow rate (0.2 bar).

The temperature is monitored; typically it rises from about 23°C to about 60°C within 60 minutes. Once the temperature reaches about 60°C, the lamps are switched off. When the temperature starts to drop, the resin kettle is transferred into a circulation oven (FED 720 available from Binder GmbH, Tuttlingen, Germany) and kept at about 60°C for about 18 hours.

After this time, the oven is switched off and the resin kettle is allowed to cool down to about 20-40°C while remaining in the oven. After that, the gel is removed and broken manually or cut with scissors into smaller pieces. The gel is grinded with a grinder (X70G available from Hermann Scharfen GmbH & Co. Maschinenfabrik KG, Witten, Germany, with Unger R70 plate system available from Licoswiss M.Liechti, Grenchen, Switzerland: 3 pre-cutter kidney plates with straight holes at 17mm diameter), put onto perforated stainless steel dishes (hole diameter 4.8 mm, 50 cm x 50 cm, 0.55 mm caliper, 50% open area, from RS; max. height of gel before drying: about 3 cm) and transferred into a circulation oven (FED 720 available from Binder GmbH, Tuttlingen, Germany) at about 105°C for about 18 hours.

The residual moisture of the dried gel is about 6.2% by weight.

In four baking trays (e.g. Kaiser 7509960, 41 x 31 x 10 cm available from W. F. Kaiser u. Co. GmbH, Diez/Lahn, Germany) an amount of the dried gel per tray is placed and an amount of deionized water (see table below) is added at once and the solution manually stirred for about 10 minutes.

|  | Tray 1 | Tray 2 | Tray 3 | Tray 4 |
|---|---|---|---|---|
| Superabsorbent polymer amount | 1500.1 g | 1500.1 g | 1500.2 g | 714.5 g |
| Water amount | 3000.0 g | 3000.1 g | 3005.0 g | 1430.6 g |

After the mixing, the wetted base polymer was kept in the trays for another 30minutes. Following, the wetted base polymer of the four trays is combined and grinded four times through a meat grinder (Grinder X70G, available from Hermann Sharfen GmbH & Co. Maschinenfabrik KG, Witten Germany, with Unger R70 plate system available from Licoswiss M.Liechti, Grenchen, Switzerland equipped with a) plate with 20 8 mm diameter holes, b) 3 shafted cutter knife and c) plate with 176 3 mm diameter holes). The feeding rate for grinding was about 300-600 g per minute. During grinding, the wetted polymer heats up and water evaporates. The wetted and grinded polymer is spread on several 50x50cm perforated stainless steel dishes (hole diameter 4.8 mm, 50 cm x 50 cm, 0.55 mm caliper, 50% open area, from RS) with a maximum gel height of about 3 cm and dried in a circulation oven (FED 720 available from Binder GmbH, Tuttlingen, Germany) at 105°C for 18 hours and subsequently for 2.5 hours at 105°C and for 14 hours in an vacuum oven (e.g. Vacutherm, VT6130 P-BL, Heraeus, Hanau, Germany, equipped with vapour trap e.g. Titan Vapor Trap, Kinetics, and/or equipped with vacuum pump e.g. Trivac®, available from Oerlikon Leybold Vacuum) at 80°C at max. about 80 mbar.

The residual moisture of the dried gel is about 3.1% by weight.

The dried gel is then ground using a centrifuge mill (ZM 200 available from Retsch GmbH, Haan, Germany, with vibratory feeder DR 100 (setting 50-60), interchangeable sieve with 1.5 mm opening settings, rotary speed 8000 rpm). The resulting base polymer particles are again dried in an oven (e.g. APT.Line FD 240 available from Binder GmbH, Tuttlingen, Germany) for 12 hours at 120°C and then sieved via a sieving machine (AS 400 control available from Retsch GmbH, Haan, Germany with sieves DIN/ISO 3310-1 at about 200-280 rpm for about for 5-10 min) to the following particle size cuts with the following yields:

| Base polymer | BP A.1 | BP A.2 | BP A.3 | BP A.4 | BP A.5 | BP A.6 |
|---|---|---|---|---|---|---|
| Particle size | <150 $\mu$m | 150-300 $\mu$m | 300-425 $\mu$m | 425-600$\mu$m | 600-710$\mu$m | >710$\mu$m |
| yield | 1026.9 g | 1217.0 g | 876.1 g | 769.9 g | 447.1 g | 789.9 g |

Base Polymer B:

A 20 000 ml resin kettle (equipped with a four-necked glass cover closed with septa, suited for the introduction of a thermometer, syringe needles) is charged with about 5388.3 g of ice (which is about 30-45% of the total amount of ice (=12149.9 g), the ice being prepared from deionized water). A magnetic stirrer, capable of mixing the whole content (when liquid), is added and stirring is started.

43.0 g of deionized water is taken to dissolve 4.516 g of "V50" (= 2,2'-azobis (N,N'-dimethyleneisobutyramidine) dihydrochloride, available from Wako Chemicals, Neuss, Germany) e.g. in a glass vessel with plastic snap-on cap. The vessel with the "V50" solution is closed and set aside in a fridge at about 4°C.

299.5 g of glacial acrylic acid (herein after referred to as "AA"; e.g. Acrylic Acid for synthesis, available from Merck KGaA, Germany) is taken from the total amount of 4000.7 g AA to dissolve 25.67 g of MBAA e.g. in a glass beaker. The beaker with the MBAA solution is covered e.g. with parafilm and set aside.

The remaining AA is added to the ice in the resin kettle while stirring is continued.

A thermometer is introduced and in total 3330.6 g of 50% NaOH solution (for analysis, available from Merck KGaA, Germany) and the remaining amount of ice (prepared from deionized water) are added subsequently in portions such that the temperature is in the range of about 15-30°C.

The MBAA solution is added to the mixture of AA, NaOH solution and ice at a temperature of about 15-30°C while stirring is continued. The beaker that contained the MBAA solution is washed 2x with deionized water in an amount of about 10% of the MBAA solution volume per wash. The wash water of both washing steps is added to the stirred mixture.

Deionized water (the remaining amount required to achieve the total amount (ice and water) of 12639.3 g minus the amount to wash the "V50" containing vessel two times with deionized water in an amount of about 10% of the "V50" solution volume per wash) is added to the stirred mixture.

Then, the resin kettle is closed, and a pressure relief is provided e.g. by puncturing two syringe needles through the septa. The solution is then purged vigorously with argon via an 80 cm injection needle at about 0.4 bar while stirring at about 400 - 1200 RPM. The argon stream is placed close to the stirrer for efficient and fast removal of dissolved oxygen.

After about min 1 hour and max 2 hours of argon purging and stirring the "V50" solution is added to the reaction mixture at a temperature of about 20-25°C via a syringe while stirring and Argon purging is continued. The vessel that contained the "V50" solution is washed two times with deionized water in an amount of about 10% of the "V50" solution volume per wash. The wash water of both washing steps is added to the stirred mixture via a syringe through the septa.

After the initiator solution is mixed with the reaction mixture, stirring and argon purging is continued for about 5 min. After that, while the reaction mixture has a temperature of about 20-25°C, two photo lamps ("ProVision 2.55 HF" available from Kaiser Fototechnik GmbH & Co.KG, Buchen, Germany, equipped with 2 lamps Osram Dulux L 55W/830, at max. intensity) are placed on either side of the vessel and switched on. The solution typically starts to become turbid or a sudden increase in viscosity is observed after about 5-20 min, typically at temperatures about room temperature. Then, the argon injection needle is raised above the surface of the gel and purging with argon is continued at a reduced flow rate (0.2 bar).

The temperature is monitored; typically it rises from about 23-24°C to about 60°C within 60 minutes. Once the temperature reaches about 60°C, the lamps are switched off. Once the temperature starts to drop, the resin kettle is transferred into a circulation oven (FED 720 available from Binder GmbH) and kept at about 60°C for about 18 hours.

After this time, the oven is switched off and the resin kettle is allowed to cool down to about 20-40°C while remaining in the oven. After that, the gel is removed and broken manually or cut with scissors into smaller pieces. The gel is grinded with a grinder (X70G available from Hermann Scharfen GmbH & Co. Maschinenfabrik KG, Witten, Germany, with Unger R70 plate system available from Licoswiss M.Liechti, Grenchen, Switzerland: 3 pre-cutter kidney plates with straight holes at 17mm diameter), put onto perforated stainless steel dishes (hole diameter 4.8 mm, 50 cm x 50 cm, 0.55 mm caliper, 50% open area, from RS; max. height of gel before drying: about 3 cm) and transferred into a circulation oven (FED 720 available from Binder GmbH) at about 120°C for about 20 hours.

The residual moisture of the dried gel is about 5.8% by weight.

In four baking trays (e.g. Kaiser 7509960, 41 x 31 x 10 cm) an amount of the dried gel per tray is placed and an amount of deionized water (see table below) is added at once and the solution manually stirred for about 10 mins.

|  | Tray 1 | Tray 2 | Tray 3 | Tray 4 |
|---|---|---|---|---|
| Superabsorbent polymer amount | 1500.1 g | 1500.4 g | 1500.1 g | 675.7 g |
| Water amount | 3000.1 g | 3002.1 g | 3000.1 g | 1353.8 g |

After the mixing, the wetted base polymer was kept in the trays for another 30minutes. Following, the wetted base polymer of the four trays is combined and grinded four times through a meat grinder (Grinder X70G available from Hermann Sharfen GmbH & Co. Maschinenfabrik KG, Germany, with Unger R70 plate system available from Licoswiss M.Liechti, Grenchen, Switzerland, equipped with a) plate with 20 8 mm diameter holes, b) 3 shafted cutter knife and c) plate with 176 3 mm diameter holes). The feeding rate for grinding was about 300-600 g per minute. During grinding, the wetted polymer heats up and water evaporates. The wetted and grinded polymer is spread on several 50x50cm perforated stainless steel dish (hole diameter 4.8 mm, 50 cm x 50 cm, 0.55 mm caliper, 50% open area, from RS) at max gel height of about 3 cm and dried in a circulation oven (FED 720 available from Binder GmbH) at 120°C for 20 hours.

The residual moisture of the dried gel is about 2.7% by weight.

The dried gel is then ground using a centrifuge mill (ZM 200 available from Retsch GmbH, Haan, Germany, with vibratory feeder DR 100 (setting 50-60), interchangeable sieve with 1.5 mm opening settings, rotary speed 8000 rpm). The milled polymer is again dried in an oven (e.g. Binder APT.Line FD 240) for 12 hours at 120°C and then sieved via a sieving machine (AS 400 control from Retsch GmbH with sieves DIN/ISO 3310-1 at about 200-280 rpm for about for 5-10 min) to the following particle size cuts with the following yields:

| Base polymer | BP B.1 | BP B.2 | BP B.3 | BP B.4 | BP B.5 | BPB.6 |
|---|---|---|---|---|---|---|
| Particle size | <150 $\mu$m | 150-300 $\mu$m | 300-425 $\mu$m | 425-600$\mu$m | 600-710$\mu$m | >710$\mu$m |
| yield | 996.4g | 1128.8 g | 822.8 g | 829.3 g | 419.2 g | 750.3 g |

Agglomerated superabsorbent polymer particles

The surface-crosslinked and agglomerated superabsorbent polymers particles SAP 1-5 were made as follows: 600.0 g the respective base polymer particles (see table) is added to a Lödige Ploughshare Laboratory Mixer, Type L5 (available from Gebrüder Lödige Maschinenbau GmbH, Paderbom, Germany) and mixed at rotary speed setting 6. The amount of Aluminium lactate solution (see table) (15w% Al lactate in deionized water (Aluminium L-lactate 95% from Sigma-Aldrich)) is added via the peristaltic pump (e.g. Ismatec MCP Standard with Tygon MHLL tube, inner diameter e.g. 1.52 mm) via a spray nozzle (spray nozzle of Mini Spray Dryer B-290 available from Buechi Labortechnik GmbH, Essen, Germany, with nozzle disc diameter 1.5 mm) at a spray pressure of about 2 bar, at a flow rate of about 4.3 g solution/min, at a starting temperature of about 23°C. After about 12.5 min the addition of Al lactate is completed. After Al solution addition is completed, the liquid hose is disconnected, cleaned and flushed with Denacol solution (solution of Denacol EX 810 (= EthyleneGlycolDiGlycidylEther = EGDGE) from Nagase in 1,2-propanediol (suitable for use as excipient, available from Merck) - see table below) and connected to the spraying unit.

The amount of Denacol EX 810 solution (see table) is added via the peristaltic pump and the spray nozzle at a spray pressure of about 2 bar at a flow rate of about 4.0 g solution/min. After the addition of Denacol EX 810 solution is completed, the liquid hose is disconnected, cleaned and flushed with deionized water and connected again to the spraying unit. After that, the amount of deionized water (see table) is added via the peristaltic pump and the spray nozzle at a spray pressure of about 2 bar at a flow rate of about 13.6 g solution/min. After the addition of deionized water is completed, the bottom outlet of the Lödige mixer is opened and the material that comes out of the bottom outlet pushed out only by the Ploughshare mixer rotation is collected and evenly distributed onto Teflon coated baking trays (e.g. Kaiser 7509960, 41 x 31 x 10 cm) into layers of about 2-3cm thickness. The baking trays are covered with aluminum foil and maintained at room temperature for about 20-24 hours. After that the covered baking trays are heated at 120°C for 2h 20 min in the oven (e.g. Binder APT.Line FD 240). After the heating time, the baking trays are taken out of the oven, the aluminium foil is cut, so about 3-6 slits of about 3 cm length and about 3 mm width are created. The samples are put under a fume hood and let cool down to room temperature. Afterwards, the samples are manually broken and sieved (with sieves DIN/ISO 3310-1 e.g. from Retsch) to get the final materials as seen in the table below.

| Superabsorbent polymer | SAP 1 | SAP 2 | SAP 3 | SAP 4 | SAP 5 | SAP 6 |
|---|---|---|---|---|---|---|
| Base polymer code | BP A.1 | BP A.1 | BP A.2 | BP A.2 | 1:1 mix of BP A.2 & B.2 | 1:1 mix of BP A.3 & B.3 |
| Al lactate solution | 72.06 | 54.03 g | 54.03 g | 72.02 g | 54.01 g | 54.03 g |
| Concentration (w%) of Denacol EX 810 solution | 24 w% | 24 w% | 24 w% | 24 w% | 16 w% | 16 w% |
| Denacol EX 810 solution | 6.05 | 6.01 g | 6.04 g | 6.00 g | 6.02 g | 6.04 g |
| Deionized water | 75.09 | 75.06 g | 75.02 g | 75.02 g | 72.08 g | 75.09 g |
| Particle size [$\mu$m] | 150-850 | 150-850 | 300-850 | 300-850 | 300-850 | 425-850 |
| yield | 460.5 g | 507.7 g | 519.1 g | 352.3 g | 423.5 g | 289.2 g |

The comparative examples SAP 7 and SAP 8 were obtained as different particle size cuts via sieving from making of SAP 5 and SAP 6, respectively (table below). Though potentially comprising some agglomerated superabsorbent polymer particles (even though the vast majority of agglomerates superabsorbent polymer particles have been sieved out), given for these examples the second mass average particle size is not at least 25% higher than the first mass average particle size, they represent comparative examples.

| Example | SAP 7 | SAP 8 |
|---|---|---|
| Base Polymer code | 1:1 mix of BP A.2 & B.2 | 1:1 mix of BP A.3 & B.3 |
| Al lactate solution | 54.01 g | 54.03 g |
| Concentration (w%) of Denacol EX 810 solution | 16 w% | 16 w% |
| Denacol EX 810 solution | 6.02 g | 6.04 g |
| Deionized water | 72.08 g | 75.09 g |
| Particle size [$\mu$m] | 150-300 | 300-425 |
| yield | 141.2 | 266.2 |

Performance:

| | T20 (s) | CRC (g/g) | FSR (g/g/s) | UPM ($10^{-7}$cm$^3$.s/g) | U20 (g/g) |
|---|---|---|---|---|---|
| SAP 3 | 117 | 24.3 | 0.55 | 71 | 27.9 |
| SAP 4 | 108 (n=4) | 23.2 | 0.55 | 90 | 25.9 (n=4) |
| SAP 5 | 104 | 25.2 | 0.59 | 49 | 29.3 |
| SAP 6 | 199 | 29.1 | 0.29 | 58 | 30.9 |

| | T20 (s) | CRC (g/g) | FSR (g/g/s) | UPM ($10^{-7}$cm$^3$.s/g) | U20 (g/g) |
|---|---|---|---|---|---|
| Example SAP 5 | 104 | 25.2 | 0.59 | 49 | 29.3 |
| Comparative Example SAP 7 | 106 | 25.0 | 0.55 | 44 | 29.7 |
| Example SAP 6 | 199 | 29.1 | 0.29 | 58 | 30.9 |
| Comparative Example SAP 8 | 182 | 28.5 | 0.28 | 53 | 30.7 |

Comparing the comparative example SAP 7 with example SAP 5 shows that SAP 5 shows better UPM performance at similar speed of absorbency (T20) and similar CRC performance than the respective comparative examples.

Comparing the comparative example SAP 8 with example SAP 6 indicates that SAP 6 shows better UPM performance (+9.4%) and better CRC performance (+2.1%) at only slightly slower speed of absorbency (T20) (-9.3%). Hence, the example SAP 6 overall performs better than the comparative example SAP 8.

Results of second mean average particle size for samples SAP 5 and SAP 6:

| | SAP 5 (n=1) | SAP 6 (n=1) |
|---|---|---|
| On 850 $\mu$m | 0.14 g | 0.21 g |
| On 800 $\mu$m | 0.11 g | 0.36 g |
| On 710 $\mu$m | 0.31 g | 0.97 g |
| On 600 $\mu$m | 1.13 g | 2.94 g |
| On 500 $\mu$m | 1.92 g | 3.34 g |
| On 425 $\mu$m | 2.28 g | 1.79 g |
| On 300 $\mu$m | 3.69 g | 0.38 g |
| On 212 $\mu$m | 0.36 g | 0 |
| On 150 $\mu$m | 0.08 g | 0 |
| On pan (0 $\mu$m) | 0 g | 0 |
| **mAvPS in $\mu$m** | **422** | **547** |

**Claims**

1. Agglomerated superabsorbent polymer particles obtained by a method comprising the steps of

   a) Providing superabsorbent polymer particles having a first mass average particle size,
   b) Mixing the superabsorbent polymer particles with a solution comprising water and a multi-valent salt having a valence of three or higher, the solution comprising from 3% to 80% by weight of the multivalent salt having a valence of three or higher;

   wherein the agglomerated superabsorbent polymer particles have a second average particle size which is at least 25% greater than the first mass average particle size.

2. The agglomerated superabsorbent polymer particles of claim 1, wherein step b) is followed by:

   c) drying the superabsorbent polymer particles, wherein the second average particle size is determined after step c).

3. The agglomerated superabsorbent polymer particles of claim 1, wherein step b) is followed by:

   c) drying the superabsorbent polymer particles, and
   d) classifying the superabsorbent polymer particles to obtain a lower particle size limit,

   wherein the second average particle size is determined after step d).

4. The agglomerated superabsorbent polymer particles any of the preceding claims, wherein the superabsorbent polymer particles are subjected to surface cross-linking after step b).

5. The agglomerated superabsorbent polymer particles of any of claims 1 to 3, wherein the superabsorbent polymer particles are subjected to surface cross-linking prior to or simultaneously with step b).

6. The agglomerated superabsorbent polymer particles of claim 4 or 5, wherein surface cross-linking includes the steps of

- Adding of one or more surface cross-linking agents
- adding water or moisture

7. The agglomerated superabsorbent polymer particles of any of the preceding claims, wherein the superabsorbent polymer particles provided in step a) are classified to obtain a defined upper particle size limit.

8. The agglomerated superabsorbent polymer particles of claim 7, wherein the superabsorbent polymer particles provided in step a) are classified to obtain a defined lower particle size limit.

9. The agglomerated superabsorbent polymer particles of claim 8, wherein the difference between the upper particle size limit and the lower particle size limit is not more 600 $\mu$m.

10. The agglomerated superabsorbent polymer particles of any of claims 7 to 9, wherein the upper particle size limit of the superabsorbent polymer particles provided in step a) is not more than 500 $\mu$m.

11. The agglomerated superabsorbent polymer particles of any of the preceding claims, wherein the superabsorbent polymer particles provided in step a) are formed of base polymer.

12. The agglomerated superabsorbent polymer particles of any of the preceding claims, wherein the multivalent salt having a valence of three or higher comprises aluminum.

13. The agglomerated superabsorbent polymer particles of any of the preceding claims, wherein the multivalent salt having a valence of three or higher comprises lactate.

14. The agglomerated superabsorbent polymer particles of any of the preceding claims, wherein, in step b), the mixing is done at a rate at least 1 g of the solution comprising water and the multi-valent salt having a valence of three or higher per kg of superabsorbent polymer per min.

15. The agglomerated superabsorbent polymer particles of any of the preceding claims, wherein the superabsorbent polymer particles provided in step a) are obtained, in this order, by the steps of:

   - providing a polyacrylic acid polymer gel; preferably by polymerizing the acrylic acid monomers at 50% to 95% neutralization,
   - submitting the gel to a first grinding step and a first drying step to obtain a base polymer particles;
   - optionally rewetting the base polymer particles;
   - submitting the base polymer particles to a second grinding step and a second drying step.

16. The agglomerated superabsorbent polymer particles of claim 15, wherein the base polymer particles have a water content of at least 0.4 g water per g of dry base polymer particles prior to being submitted to the second grinding step and the second drying step.

17. The agglomerated superabsorbent polymer particles according to one of the preceding claims, wherein the agglomerated superabsorbent polymer particles have a time to reach an uptake of 20 g/g (T20) of less than 240s, preferably from at least 40s and less than 240s, as measured according to the K(t) test method described herein.

18. The agglomerated superabsorbent polymer particles according to one of the preceding claims, wherein the agglomerated superabsorbent polymer particles have an UPM value of from $40.10^{-7}$ cm$^3$.s/g to $500.10^{-7}$ cm$^3$.s/g and/or a CRC value of from 18 g/g to 40 g/g.

19. An absorbent article comprising the agglomerated superabsorbent polymer particles according to one of the preceding claims.

20. A diaper or pant comprising a topsheet, a backsheet and an absorbent core disposed there between, wherein the absorbent core comprises at least 70%, or at least 80%, or at least 90% or 100% of superabsorbent polymer particles by weight of the absorbent material, wherein the superabsorbent polymer particles comprise at least 10%, or at least 20% or at least 30% or at least 50% by weight of the agglomerated superabsorbent polymer particles of any of claims 1 to 18.

**Patentansprüche**

1. Agglomerierte Superabsorberpolymerteilchen, erhalten durch ein Verfahren, umfassend die folgenden Schritte

   a) Bereitstellen von Superabsorberpolymerteilchen mit einer ersten massegemittelten Teilchengröße,
   b) Mischen der Superabsorberpolymerteilchen mit einer Lösung umfassend Wasser und ein mehrwertiges Salz mit einer Wertigkeit von drei oder höher, wobei die Lösung zu 3 Gew.-% bis 80 Gew.-% das mehrwertige Salz mit einer Wertigkeit von drei oder höher umfasst;

   wobei die agglomerierten Superabsorberpolymerteilchen eine zweite durchschnittliche Teilchengröße aufweisen, die um wenigstens 25 % größer ist als die erste massegemittelte Teilchengröße.

2. Agglomerierte Superabsorberpolymerteilchen nach Anspruch 1, wobei Schritt b) gefolgt wird von:

   c) Trocknen der Superabsorberpolymerteilchen, wobei die zweite durchschnittliche Teilchengröße nach Schritt c) bestimmt wird.

3. Agglomerierte Superabsorberpolymerteilchen nach Anspruch 1, wobei Schritt b) gefolgt wird von:

   c) Trocknen der Superabsorberpolymerteilchen, und
   d) Klassieren der Superabsorberpolymerteilchen, um eine geringere untere Teilchengrößengrenze zu erhalten,

   wobei die zweite durchschnittliche Teilchengröße nach Schritt d) bestimmt wird.

4. Agglomerierte Superabsorberpolymerteilchen nach einem der vorstehenden Ansprüche, wobei die Superabsorberpolymerteilchen nach Schritt b) einer Oberflächenvernetzung unterzogen werden.

5. Agglomerierte Superabsorberpolymerteilchen nach einem der Ansprüche 1 bis 3, wobei die Superabsorberpolymerteilchen vor oder gleichzeitig mit Schritt b) einer Oberflächenvernetzung unterzogen werden.

6. Agglomerierte Superabsorberpolymerteilchen nach Anspruch 4 oder 5, wobei die Oberflächenvernetzung folgende Schritte einschließt

   - Zugeben eines oder mehrerer oberflächenvernetzender Mittel
   - Zugeben von Wasser oder Feuchtigkeit

7. Agglomerierte Superabsorberpolymerteilchen nach einem der vorstehenden Ansprüche, wobei die Superabsorberpolymerteilchen, die in Schritt a) bereitgestellt werden, klassiert werden, um eine definierte obere Teilchengrößengrenze zu erhalten.

8. Agglomerierte Superabsorberpolymerteilchen nach Anspruch 7, wobei die Superabsorberpolymerteilchen, die in Schritt a) bereitgestellt werden, klassiert werden, um eine definierte untere Teilchengrößengrenze zu erhalten.

9. Agglomerierte Superabsorberpolymerteilchen nach Anspruch 8, wobei die Differenz zwischen der oberen Teilchengrößengrenze und der unteren Teilchengrößengrenze nicht größer als 600 $\mu$m ist.

10. Agglomerierte Superabsorberpolymerteilchen nach einem der Ansprüche 7 bis 9, wobei die obere Teilchengrößengrenze der Superabsorberpolymerteilchen, die in Schritt a) bereitgestellt werden, nicht größer als 500 $\mu$m ist.

11. Agglomerierte Superabsorberpolymerteilchen nach einem der vorstehenden Ansprüche, wobei die Superabsorberpolymerteilchen, die in Schritt a) bereitgestellt werden, aus Basispolymer gebildet sind.

12. Agglomerierte Superabsorberpolymerteilchen nach einem der vorstehenden Ansprüche, wobei das mehrwertige Salz mit einer Wertigkeit von drei oder höher Aluminium umfasst.

13. Agglomerierte Superabsorberpolymerteilchen nach einem der vorstehenden Ansprüche, wobei das mehrwertige Salz mit einer Wertigkeit von drei oder höher Lactat umfasst.

**14.** Agglomerierte Superabsorberpolymerteilchen nach einem der vorstehenden Ansprüche, wobei in Schritt b) das Mischen bei einer Rate von mindestens 1 g der Lösung, die Wasser und das mehrwertige Salz mit einer Wertigkeit von drei oder höher umfasst, pro kg des Superabsorberpolymers pro Minute erfolgt.

**15.** Agglomerierte Superabsorberpolymerteilchen nach einem der vorstehenden Ansprüche, wobei die Superabsorberpolymerteilchen, die in Schritt a) bereitgestellt werden, in der aufgeführten Reihenfolge durch folgende Schritte erhalten werden:

- Bereitstellen eines Polyacrylsäure-Polymergels; vorzugsweise durch Polymerisieren der Acrylsäuremonomere bei 50 % bis 95 % Neutralisation,
- Unterziehen des Gels einem ersten Mahlschritt und einem ersten Trocknungsschritt, um Basispolymerteilchen zu erhalten;
- wahlweise Wiederbenetzen der Basispolymerteilchen;
- Unterziehen der Basispolymerteilchen einem zweiten Mahlschritt und einem zweiten Trocknungsschritt.

**16.** Agglomerierte Superabsorberpolymerteilchen nach Anspruch 15, wobei die Basispolymerteilchen einen Wassergehalt von mindestens 0,4 g Wasser pro g der trockenen Basispolymerteilchen aufweisen, bevor sie dem zweiten Mahlschritt und dem zweiten Trocknungsschritt unterzogen werden.

**17.** Agglomerierte Superabsorberpolymerteilchen nach einem der vorstehenden Ansprüche, wobei die agglomerierten Superabsorberpolymerteilchen eine Zeit bis zum Erreichen einer Aufnahme von 20 g/g (T20) von weniger als 240 s, vorzugsweise von mindestens 40 s und weniger als 240 s, gemessen nach dem hierin beschriebenen Prüfverfahren K(t), aufweisen.

**18.** Agglomerierte Superabsorberpolymerteilchen nach einem der vorstehenden Ansprüche, wobei die agglomerierten Superabsorberpolymerteilchen einen UPM-Wert von $40,10^{-7}$ cm$^3$.s/g bis $500,10^{-7}$ cm$^3$.s/g und/oder einen CRC-Wert von 18 g/g bis 40 g/g aufweisen.

**19.** Absorptionsartikel, der die agglomerierten Superabsorberpolymerteilchen nach einem der vorstehenden Ansprüche umfasst.

**20.** Windel oder Hose, umfassend eine Oberschicht, eine Unterschicht und einen Absorptionskern, der dazwischen angeordnet ist, wobei der Absorptionskern bezogen auf das Gewicht des Absorptionsmaterials zu mindestens 70 Gew.-% oder zu mindestens 80 Gew.-% oder zu mindestens 90 Gew.-% oder zu 100 Gew.-% Superabsorberpolymerteilchen umfasst, wobei die Superabsorberpolymerteilchen zu mindestens 10 Gew.-% oder zu mindestens 20 Gew.-% oder zu mindestens 30 Gew.-% oder zu mindestens 50 Gew.-% die agglomerierten Superabsorberpolymerteilchen nach einem der Ansprüche 1 bis 18 umfassen.

**Revendications**

**1.** Particules de polymère superabsorbant agglomérées obtenues par un procédé comprenant les étapes de

a) fourniture de particules de polymère superabsorbant ayant une première taille de particule moyenne en masse,
b) mélange des particules de polymère superabsorbant avec une solution comprenant de l'eau et un sel multivalent ayant une valence de trois ou plus, la solution comprenant de 3 % à 80 % en poids du sel multivalent ayant une valence de trois ou plus ;

dans lequel les particules de polymère superabsorbant agglomérées ont une deuxième taille de particule moyenne qui est au moins 25 % supérieure à la première taille de particule moyenne en masse.

**2.** Particules de polymère superabsorbant agglomérées selon la revendication 1, dans lesquelles l'étape b) est suivie par :

c) le séchage des particules de polymère superabsorbant, dans lesquelles la deuxième taille de particule moyenne est déterminée après l'étape c).

**3.** Particules de polymère superabsorbant agglomérées selon la revendication 1, dans lesquelles l'étape b) est suivie par :

c) le séchage des particules de polymère superabsorbant, et
d) la classification des particules de polymère superabsorbant pour obtenir une limite inférieure de taille de particule,

dans lesquelles la deuxième taille moyenne de particule est déterminée après l'étape d).

4. Particules de polymère superabsorbant agglomérées selon l'une quelconque des revendications précédentes, dans lesquelles les particules de polymère superabsorbant sont soumises à une réticulation de surface après l'étape b).

5. Particules de polymère superabsorbant agglomérées selon l'une quelconque des revendications 1 à 3, où les particules de polymère superabsorbant sont soumises à une réticulation de surface avant l'étape b) ou simultanément à l'étape b).

6. Particules de polymère superabsorbant agglomérées selon la revendication 4 ou 5, dans lesquelles la réticulation de surface inclut les étapes de :

- ajout d'un ou de plusieurs agents de réticulation de surface
- ajout d'eau ou d'humidité

7. Particules de polymère superabsorbant agglomérées selon l'une quelconque des revendications précédentes, où les particules de polymère superabsorbant fournies à l'étape a) sont classées pour obtenir une limite supérieure définie de taille de particule.

8. Particules de polymère superabsorbant agglomérées selon la revendication 7, où les particules de polymère superabsorbant fournies à l'étape a) sont classées pour obtenir une limite inférieure définie de taille de particule.

9. Particules de polymère superabsorbant agglomérées selon la revendication 8 dans lesquelles la différence entre la limite supérieure de taille de particule et la limite inférieure de taille de particule ne dépasse pas 600 $\mu$m.

10. Particules de polymère superabsorbant agglomérées selon l'une quelconque des revendications 7 à 9, dans lesquelles la limite supérieure de taille de particule des particules de polymère superabsorbant fournies à l'étape a) ne dépasse pas 500 $\mu$m.

11. Particules de polymère superabsorbant agglomérées selon l'une quelconque des revendications précédentes, où les particules de polymère superabsorbant fournies à l'étape a) sont formées de polymère de base.

12. Particules de polymère superabsorbant agglomérées selon l'une quelconque des revendications précédentes, dans lesquelles le sel multivalent ayant une valence de trois ou plus comprend de l'aluminium.

13. Particules de polymère superabsorbant agglomérées selon l'une quelconque des revendications précédentes, dans lesquelles le sel multivalent ayant une valence de trois ou plus comprend du lactate.

14. Particules de polymère superabsorbant agglomérées selon l'une quelconque des revendications précédentes, dans lesquelles, à l'étape b), le mélange est effectué à une vitesse d'au moins 1 g de la solution comprenant de l'eau et le sel polyvalent ayant une valence de trois ou plus par kg de polymère superabsorbant par minute.

15. Particules de polymère superabsorbant agglomérées selon l'une quelconque des revendications précédentes, où les particules de polymère superabsorbant fournies à l'étape a) sont obtenues, dans cet ordre, par les étapes de :

- fourniture d'un gel de polymère d'acide polyacrylique ; de préférence par polymérisation des monomères d'acide acrylique à une neutralisation de 50 % à 95 %,
- soumission du gel à une première étape de broyage et une première étape de séchage pour obtenir des particules de polymère de base ;
- facultativement mouillage à nouveau des particules de polymère de base ;
- soumission des particules de polymère de base à une deuxième étape de broyage et une deuxième étape de séchage.

**16.** Particules de polymère superabsorbant agglomérées selon la revendication 15, où les particules de polymère de base ont une teneur en eau d'au moins 0,4 g d'eau par g de particules de polymère de base sèches avant d'être soumises à la deuxième étape de broyage et à la deuxième étape de séchage.

**17.** Particules de polymère superabsorbant agglomérées selon l'une des revendications précédentes, où les particules de polymère superabsorbant agglomérées ont un temps pour atteindre une absorption de 20 g/g (T20 inférieur à 240 s, de préférence d'au moins 40 s et inférieur à 240 s, tel que mesuré selon le procédé de test K(t) décrit ici.

**18.** Particules de polymère superabsorbant agglomérées selon l'une des revendications précédentes, où les particules de polymère superabsorbant agglomérées ont une valeur PMU allant de $40.10^{-7}$ $cm^3$.s/g à $500.10^{-7}$ $cm^3$.s/g et/ou une valeur CRC allant de 18 g/g à 40 g/g.

**19.** Article absorbant comprenant les particules de polymère superabsorbant agglomérées selon l'une des revendications précédentes.

**20.** Couche ou culotte comprenant une feuille de dessus, une feuille de fond et une âme absorbante disposée entre elles, dans laquelle l'âme absorbante comprend au moins 70 %, ou au moins 80 %, ou au moins 90 % ou 100 % de particules de polymère superabsorbant en poids du matériau absorbant, dans laquelle les particules de polymère superabsorbant comprennent au moins 10 %, ou au moins 20 % ou au moins 30 % ou au moins 50 % en poids des particules de polymère superabsorbant agglomérées de l'une quelconque des revendications 1 à 18.

**Fig. 1**

**Fig. 2**

EP 2 944 376 B1

Figure 3

Figure 4

**EP 2 944 376 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7776984 B
- WO 2009095810 A
- WO 2005012406 A
- US 4340706 A
- US 5849816 A
- US 20090192035 A
- US 20090258994 A
- US 20100068520 A
- WO 2006083584 A

- EP 2535027 A1
- WO 200059430 A
- WO 9510996 A
- US 5700254 A
- WO 02067809 A
- US 7786341 B
- EP 149880 A, Kwok
- US 20030105190 A, Diehl

**Non-patent literature cited in the description**

- Absorbency. Elsevier, 1982, 42-43

- **J.M. COULSON ; J.F. RICHARDSON.** Chemical Engineering. Pergamon Press, 1978, vol. II, 122-127